# EUROPEAN PATENT APPLICATION

(11) **EP 4 537 843 A1**
(43) Date of publication of application: **16.04.2025**
(21) Application number: 23834368.5
(22) Date of filing: 09.02.2023
(51) Int. Cl.: A61K 39/395, A61K 36/258, A61P 35/00

(54) **USE OF GINSENOSIDE IN COMBINATION WITH PD-1 BLOCKER IN PREPARING MEDICAMENT AGAINST HEAD AND NECK SQUAMOUS CELL CARCINOMA**

(30) Priority: 08.07.2022 CN 202210806755
(71) Applicant: Institute of Medicinal Plant Development, Chinese Academy of Medical Sciences, Beijing 100193 (CN)
(72) Inventor: SUN, Xiaobo, Beijing 100193 (CN); XING, Xiaoyan, Beijing 100193 (CN); QIANG, Weijie, Beijing 100193 (CN); WANG, Yixin, Beijing 100193 (CN)
(74) Representative: Schlich
(86) International application number: PCT/CN2023/075142
(87) International publication number: WO 2024/007583

(57) **Abstract**

The present application provides use of a ginsenoside in combination with a PD-1 blocker in preparing a medicament against head and neck squamous cell carcinoma and a corresponding pharmaceutical composition, According to the present application, the combined use of ginsenoside Rg3 and a metabolite Rh2 thereof with the PD-1 blocker can enhance the inhibitory effect of the PD-1 blocker on head and neck squamous cell carcinoma, enhance the infiltration level of immune cells in tumor tissues, and enhance the killing effect of lymphocytes on tumor cells, providing a new medication option for the treatment of head and neck squamous cell carcinoma. The present application has wide medical application prospects.

## Description

### CROSS REFERENCE TO RELATED APPLICATIONS

The present application claims priority to Chinese Patent Application No. 202210806755.5, entitled "USE OF GINSENOSIDE IN COMBINATION WITH PD-1 BLOCKER IN PREPARING MEDICAMENT AGAINST HEAD AND NECK SQUAMOUS CELL CARCINOMA", and filed to the China National Intellectual Property Administration on July 8, 2022, the entire contents of which are incorporated herein by reference.

### TECHNICAL FIELD

The present application belongs to the technical field of medicament for treating head and neck squamous cell carcinoma. Specifically, the present application provides use of a ginsenoside Rh2 or ginsenoside Rg3 in combination with a PD-1 blocker in preparing a medicament for treating head and neck squamous cell carcinoma.

### BACKGROUND

Head and neck squamous cell carcinoma (referred to as HNSCC) is one of the most common malignant tumors in the world. There are more than 650,000 new cases and 330,000 deaths every year, which are characterized by high mortality and easy recurrence; see Bray F, Ferlay J, Soerjomataram I, Siegel RL, Torre LA, Jemal A. Global cancer statistics 2018: GLOBOCAN estimates of incidence and mortality worldwide for 36 cancers in 185 countries. CA Cancer J Clin. 2018; 68:394-424. This is a highly invasive heterologous disease with a special anatomic site and a variety of complex types, which seriously affects the patient's appearance, chewing, smell and other basic physiological functions and quality of life. Immunotherapy is a treatment method that promotes the immune system to recognize and eliminate tumor cells by restoring the body's normal immune response. It is considered the most promising method for treating advanced or invasive cancer, with PD-1 blockers being the most widely studied and applied. Although immunotherapy can significantly improve the treatment effect of patients with head and neck squamous cell carcinoma, the objective response rate is only about 20%, and only a small number of patients can benefit from it. Studies have shown that combination immunotherapy can significantly improve the tumor response rate and reduce the risk of disease progression, thereby prolonging overall survival and improving patients' quality of life. Currently, common combination immunotherapy regimens include: PD-1 blockers in combination with chemotherapy, PD-1 blockers in combination with radiotherapy, PD-1 blockers in combination with targeted medicaments, PD-1 blocker in combination with other immunotherapy.

Studies have shown that although the above combination immunotherapy regimens can improve treatment effects, they often lead to more severe side effects than single-agent treatments. The probability of experiencing grade III or higher side effects when PD-1 blockers are used in combination with chemotherapy, radiotherapy, targeted therapy, and immunotherapy is 12.4%, 68.3%, 47.3%, and 35.9%, respectively. The most common side effects are: dysphagia (30.0%), anemia (45.4%), fatigue (34.3%), and fatigue (26.4%); see Zhou X, Yao Z, Bai H, Duan J, Wang Z, Wang X, Zhang X, Xu J, Fei K, Zhang Z, Tan F, Xue Q, Gao S, et al. Treatment-related adverse events of PD-1 and PD-L1 inhibitor-based combination therapies in clinical trials: a systematic review and meta-analysis. Lancet Oncol. 2021; 22(9): 1265-1274.

Traditional Chinese medicine has certain advantages in regulating the body's immune function and reducing toxic effects. Traditional Chinese medicine with immunomodulatory effects has good potential to be used in combination with immunotherapy. Ginseng is the dried root of Panax ginseng, a plant in the Panx L. genus of Araliaceae family. It is a representative traditional Chinese medicine with immune-regulating effects. It has the functions of tonifying vitality, rejuvenating pulse and fixing prolapse, nourishing the spleen and lungs, producing body fluids and calming the nerves. As the main active ingredient in ginseng, ginsenosides have been widely studied for their anti-tumor and immune modulation effects. Ginsenosides can undergo metabolic transformation in the body, and their anticancer activity and bioavailability are increased. Studies have shown that ginsenoside Rh2 is the main metabolite of ginsenoside Rg3, with its oral bioavailability increased by 5 times and its content increased by 50 times; see Yang, L., Zou, H., Gao, Y., Luo, J., Xie, X., Meng, W., Zhou, H., & Tan, Z. (2020). Insights into gastrointestinal microbiota-generated ginsenoside metabolites and their bioactivities. Drug metabolism reviews, 52(1), 125-138; Zhao C, Sun R, Cao B, et al. An in vitro metabolic system of gut flora and the metabolism of ginsenoside Rg3 and cholic acid. Eur J Drug Metab Pharmacokinet. 2014; 39(2): 129-137. Ginsenosides Rg3 and ginsenoside Rh2 are considered to be valuable for further development and utilization, and have the potential to enhance the anti-tumor efficacy of immunotherapy.

### SUMMARY OF THE INVENTION

In order to further enrich the combination therapy methods of PD-1 blockers and develop the application potential of ginsenosides, the present application has studied and developed a combination therapy of ginsenosides and PD-1 blockers for the treatment of head and neck squamous cell carcinoma, so as to fully exert the synergistic effect of medicaments and reduce toxicity, providing a safer and more effective medicament combination and usage for clinical treatment of head and neck squamous cell carcinoma.

In an aspect, the present application provides use of ginsenosides in combination with a PD-1 blocker in preparing a medicament against head and neck squamous cell carcinoma.

In another aspect, the present application provides use of a ginsenoside in preparing a synergist for enhancing the therapeutic effect of the PD-1 blocker on head and neck squamous cell carcinoma.

In another aspect, the present application provides a pharmaceutical composition for treating head and neck squamous cell carcinoma, wherein the pharmaceutical composition comprises a ginsenoside and a PD-1 blocker.

In another aspect, the present application provides a method for treating head and neck squamous cell carcinoma, comprising administering a ginsenoside and a PD-1 blocker to a subject in need thereof.

Optionally, the ginsenoside is ginsenoside Rh2 or ginsenoside Rg3.

Optionally, the PD-1 blocker is an anti-PD-1 antibody, preferably pembrolizumab.

Optionally, the ginsenoside enhances the inhibitory effect of the PD-1 blocker on head and neck squamous cell carcinoma.

Optionally, the ginsenoside enhances the infiltration level of immune cells in tumor tissue.

Optionally, the ginsenoside and the PD-1 blocker enhance the killing effect of lymphocytes on tumor cells.

Optionally, the ginsenoside is in an oral dosage form and the PD-1 blocker is in an injection dosage form.

Optionally, the clinical dose of ginsenoside in the pharmaceutical composition is 1.5 mg/kg to 2.0 mg/kg.

Optionally, the pharmaceutical composition further comprises other medicament for treating or auxiliary treating head and neck squamous cell carcinoma. The medicament for treating or auxiliary treating head and neck squamous cell carcinoma is ganoderma lucidum polysaccharide and/or astragalus polysaccharide.

Optionally, the pharmaceutical composition further comprises a pharmaceutically acceptable excipient.

In the present application, PD-1 refers to Programmed death-1 and programmed cell death receptor 1; these names can be used interchangeably to express the same meaning.

Various types of ginsenosides that have been identified and are under studied may be used in the present application, preferably ginsenoside Rh2; ginsenoside Rh2 is a metabolite of ginsenoside Rg3, and the pharmacological activities of ginsenoside Rh2 and ginsenoside Rg3 should be similar, so ginsenoside Rg3 should also be able to be used in combination with a PD-1 blocker to achieve similar effects as ginsenoside Rh2. The ginsenosides and the PD-1 blocker in the medicine of the present application can be prepared in the same pharmaceutical composition, or in different pharmaceutical compositions and used in combination. Available dosage forms of ginsenosides and PD-1 blockers include but are not limited to oral preparations, such as tablets, capsules, oral liquids; injections, such as aqueous injections, powder injections, etc. Those skilled in the art can select dosage forms based on common pharmaceutical knowledge and the available dosage forms of existing PD-1 blockers.

Depending on the dosage form used, pharmaceutically acceptable excipients that can be used in the pharmaceutical composition of the present application include but are not limited to solvents, cosolvents, surfactants, pH adjusters, osmotic pressure regulators, stabilizers, dispersants, fillers, adhesives, coating agents, capsule shells, flavoring agents, etc.

Other medicaments used for the treatment or adjuvant treatment of head and neck squamous cell carcinoma include various known and researched Chinese and Western medicaments, such as ganoderma lucidum polysaccharide, astragalus polysaccharide and other natural products with immune-enhancing effects, other chemotherapy medicaments, targeted medicaments, etc.

### Beneficial effects:

This application found that the use of ginsenoside Rh2 in combination with PD-1 blockers can enhance the inhibitory effect of PD-1 blockers on head and neck squamous cell carcinoma, enhance the infiltration level of immune cells in tumor tissues, and enhance the killing effect of lymphocytes on tumor cells, providing a new medication option for the treatment of head and neck squamous cell carcinoma, a difficult cancer.

### DESCRIPTION OF THE DRAWINGS

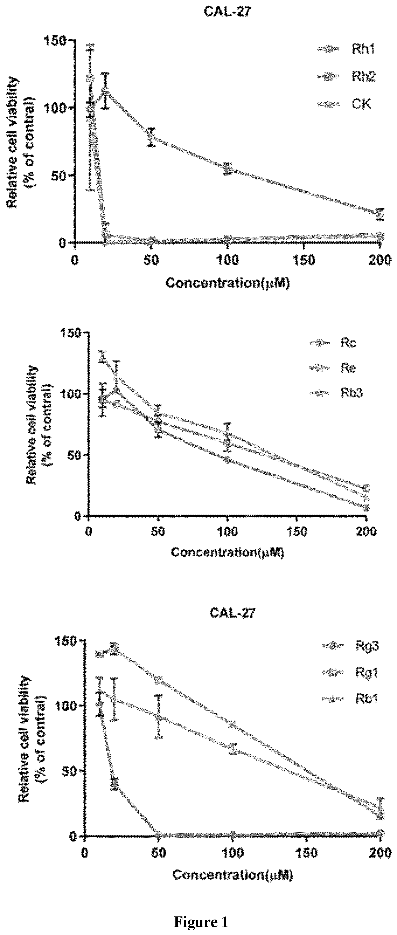

Figure 1 shows the effects of 9 ginsenoside monomers on the proliferation of tongue squamous cell carcinoma Cal-27 cells;

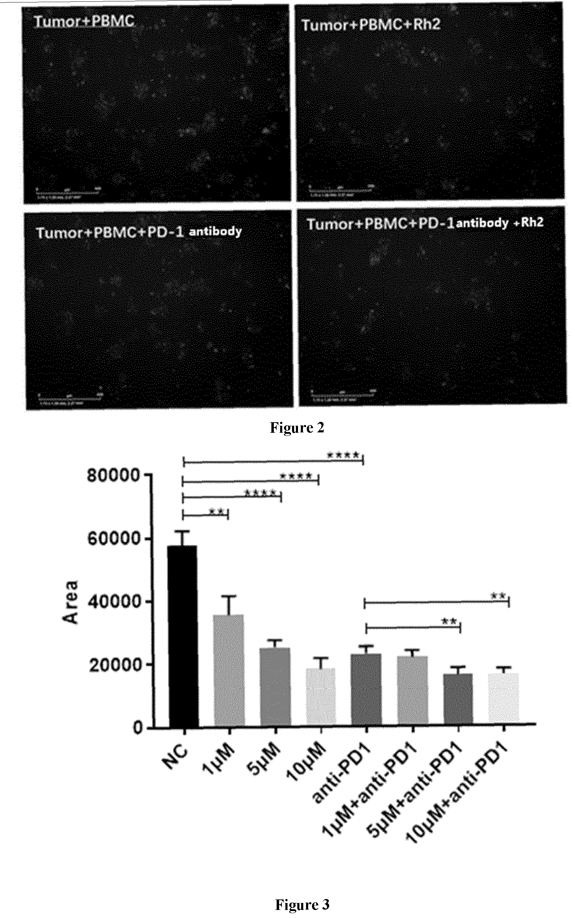

Figure 2 is a diagram showing the effect of ginsenoside Rh2 in combination with anti-PD-1 antibody on tumor cells. Tumor cells are labeled with red fluorescent reagent, so variations in the number of tumor cells are expressed as fluorescence area;
Figure 3 shows the effects of ginsenoside Rh2 and anti-PD-1 antibody on the number of tumor cells. Cal-27 cells and PBMC are co-cultured with or without ginsenoside Rh2 or anti-PD-1 antibody respectively. The concentrations of ginsenoside Rh2 are 1µM, 5µM and 10µM respectively, and the concentration of anti-PD-1 antibody is 5µg/mL. **P<0.01, ****P<0.0001;

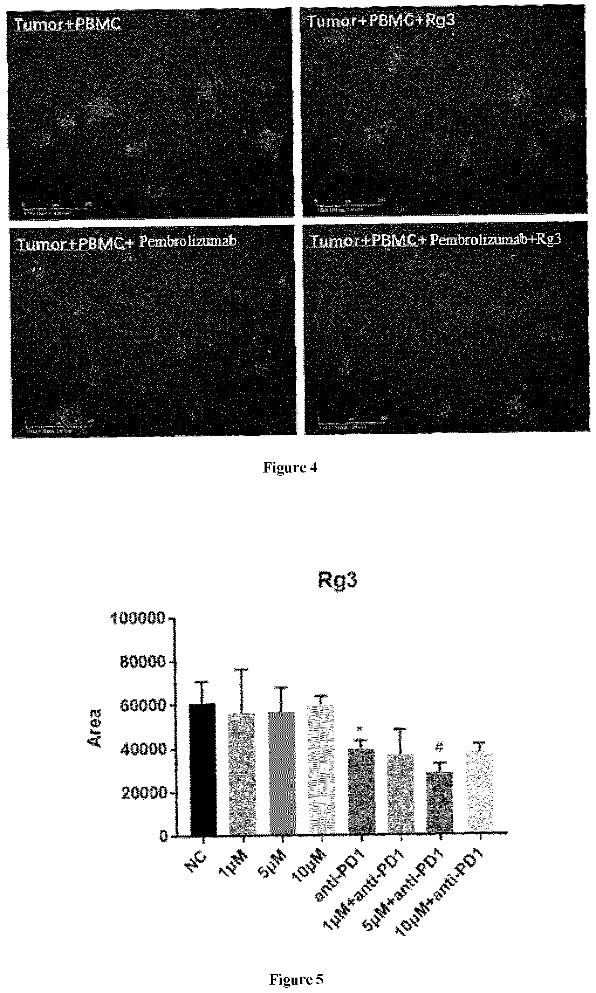

Figure 4 is a diagram showing the effect of ginsenoside Rg3 in combination with anti-PD-1 antibody on tumor cells. Tumor cells are labeled with red fluorescent reagent, so variations in the number of tumor cells are expressed as fluorescence area;
Figure 5 shows the effects of ginsenoside Rg3 and anti-PD-1 antibody on the number of tumor cells. Cal-27 cells and PBMC are co-cultured with or without ginsenoside Rg3 or anti-PD-1 antibody respectively. The concentrations of ginsenoside Rg3 are 1µM, 5µM and 10µM respectively, and the concentration of anti-PD-1 antibody is 5µg/mL. *P<0.05 vs NC group; #P<0.05 vs anti-PD-1 antibody group;

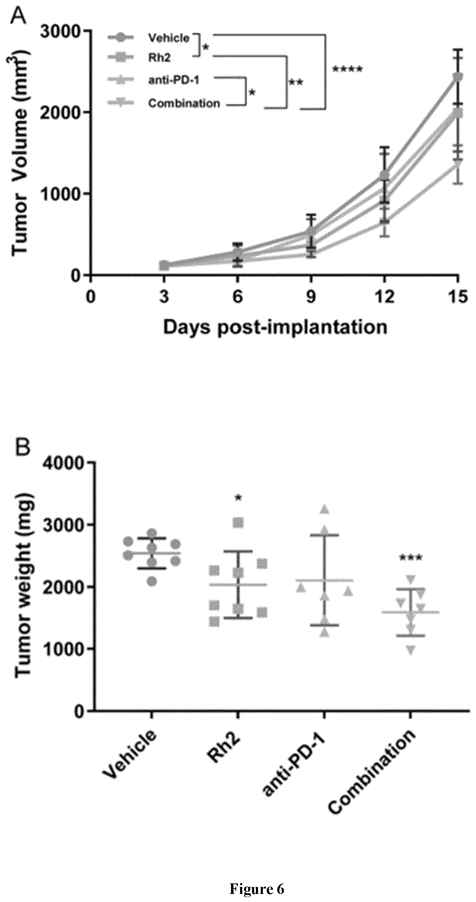

Figure 6 shows the effects of ginsenoside Rh2 and anti-PD-1 antibody on tumor growth. (A) Effects of ginsenoside Rh2 and anti-PD-1 antibody on tumor volume in mice. (B) Effects of ginsenoside Rh2 and anti-PD-1 antibody on tumor weight in mice. The dose of ginsenoside Rh2 is 15 mg/kg, and the dose of anti-PD-1 antibody is 200 µg/mice. *P<0.05, **P<0.01, ***P<0.001, ****P<0.0001;

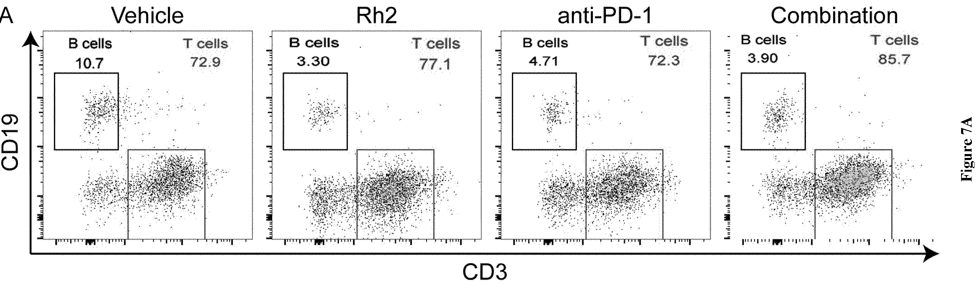

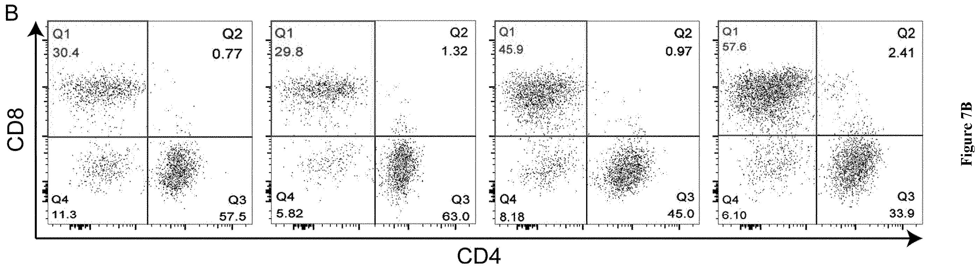

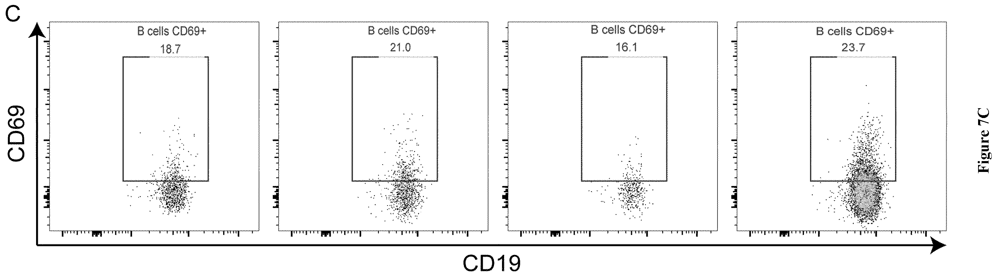

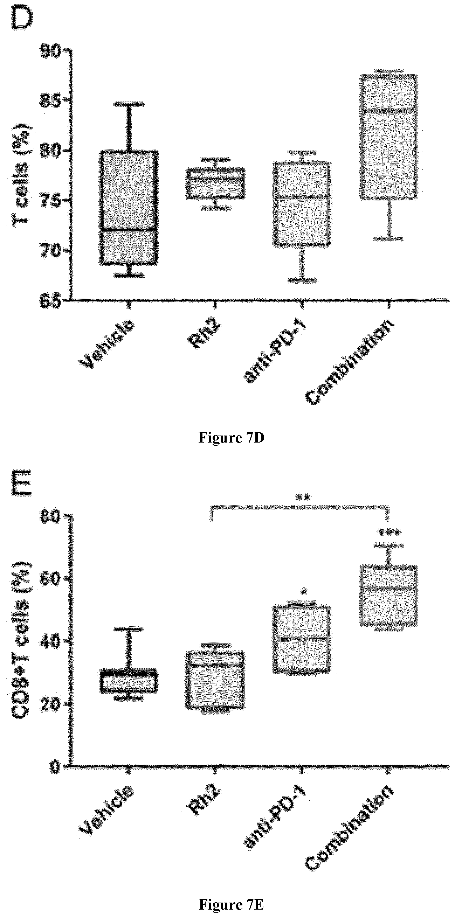

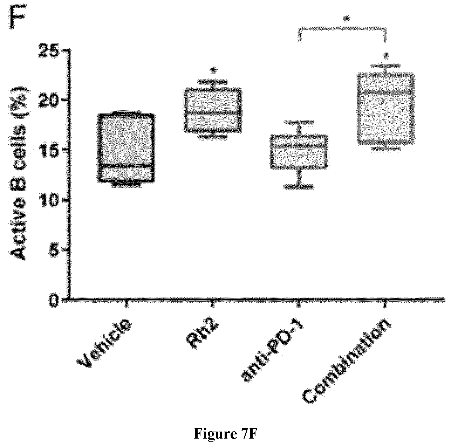

Figure 7 shows the effects of ginsenoside Rh2 and anti-PD-1 antibody on infiltration level of immune cell in mice tumor tissues. (A) (D) Effect of ginsenoside Rh2 and anti-PD-1 antibody on T cells; (B) (E) Effect of ginsenoside Rh2 and anti-PD-1 antibody on CD8⁺ T cells; (C) (F) Effects of ginsenoside Rh2 and anti-PD-1 antibody on B cell activation. *P<0.05, **P<0.01.

### DETAILED DESCRIPTION

The present application will be described in detail below with reference to specific examples, but the protection scope of the present application is not limited to the following examples.

The anti-PD-1 antibody used in the following experiments is pembrolizumab; the tumor cells are Cal-27 cells or SCC-7 cells; the lymphocytes are lymphocytes induced by PBMC stimulated by PHA.

### Example 1 Effects of 9 ginsenoside monomers on the proliferation of tongue squamous cell carcinoma Cal-27 cells

### 1. Experimental method

Cells in good growth state were counted and inoculated into 96-well plates with 100µL per well according to a certain cell density. After the cells adherence to the wall overnight, a medicament solution was added to intervene the cells. After 48 h of medicament treatment, CCK-8 reagent was added, 10 µL per well. After incubation for 2 h, the culture plates were taken out and detected at 450 nm wavelength of the enzymograph.

### 2. Experimental result

As shown in Figure 1, ginsenosides Rh1, Rc, Re, Rb3, Rg1 and Rb1 had concentration-dependent inhibitory effects on Cal-27 cells, and the inhibitory effects were weak at low concentration and strong at high concentration, with IC50 all around 100µM. In addition, ginsenosides CK, Rh2 and Rg3 had the most significant inhibitory effect on Cal-27 cells, and had a good inhibitory effect at low concentration.

### Example 2 Effect of ginsenoside Rh2 or ginsenoside Rg3 in combination with anti-PD-1 antibody on lymphocyte killing in vitro

### 1. Experimental method

### 1.1 Determination of the effect-to-target ratio of lymphocyte and tumor cell co-culture system

Cal-27 cells in good growth state were digested and centrifuged, appropriate amount of cell suspension was taken and placed in a new centrifuge tube, incubated with the target red label reagent for 20min, washed and centrifuged with serum-free culture solution, then plated, and placed in an incubator for overnight cultivation to make them adhere to the wall. PBMC stimulated by PHA was added into the well plate in ratios of 1:1, 5:1, 10:1 and 20:1, and Incucyte real-time dynamic cell imaging system was used to observe the killing effect of lymphocytes on tumor cells under different target ratio conditions.

### 1.2 Detection of the killing effect of lymphocytes on tumor cells

Cal-27 cells in good growth statu were digested and centrifuged, incubated with red target labeling reagent for 20min, and washed with serum-free culture solution. After the cells were adherence to the wall overnight, the PBMC stimulated by PHA was added to the well plate according to the effect-to-target ratio of 5:1 and co-cultured with Cal-27 cells. Ginsenoside Rh2 or ginsenoside Rg3 of medicament solution with different concentrations were prepared, and they and 5µg/mL human anti-PD-1 antibody were added into the well plate for incubation, and were divided into control group, ginsenoside alone group, anti-PD-1 antibody alone group, ginsenoside and anti-PD-1 antibody combination group. The culture plates were placed into Incucyte real-time dynamic cell imaging analysis system for real-time monitoring.

### 2. Experimental result

In the co-culture experiment, we labeled tumor cells with red fluorescent reagent (Figure 2 and Figure 4), so the variations in the number of tumor cells can be expressed by the area of red fluorescence. As shown in Figure 3, compared with the PBMC control group, ginsenoside Rh2 with 1 µM, 5 µM and 10 µM can reduce the number of Cal-27 cells, indicating that ginsenoside Rh2 can dose-dependently promote the killing effect of lymphocytes on tumor cells; Anti-PD-1 antibody can also significantly promote the killing effect of lymphocytes. Compared with anti-PD-1 antibody alone, ginsenoside Rh2 in combination with anti-PD-1 antibody at 5 µM and 10 µM can further enhance the killing effect of lymphocytes, and the results are statistically significant (P<0.05). As shown in Figure 5, compared with the PBMC control group, the anti-PD-1 antibody can significantly promote the killing effect of lymphocytes; compared with the anti-PD-1 antibody alone, ginsenoside Rg3 at 5 µM in combination with the anti-PD-1 antibody can significantly enhance the killing effect of lymphocytes. The above results show that both ginsenoside Rh2 or ginsenoside Rg3 and anti-PD-1 antibody can promote the killing effect of lymphocytes on head and neck squamous cell carcinoma cells, and the combination of these two medicaments with anti-PD-1 antibody can significantly enhance the killing ability of lymphocytes.

### Example 3 Study on the efficacy of ginsenoside Rh2 in combination with anti-PD-1 antibody in head and neck squamous cell carcinoma-bearing mice

### 1. Experimental animal

SPF grade C57 mice, 6 weeks old, female, were purchased from Beijing Charles River Laboratories Animal Company. They were reared in the Animal Center of the CHINESE ACADEMY OF MEDICAL SCIENCES INSTITUTE OF MEDICINAL PLANT DEVELOPMENT. The experimental facilities comply with barrier environmental standards, with a temperature of 20°C to 26°C, a relative humidity of 40% to 70%, and light and dark alternating for 12 hours each. Mice were raised in standard boxes, with 5 mice per cage. During the raising process, the animals were allowed to eat and move freely.

### 2. Experimental method

### 2.1 Grouping and administration

The mice were randomly divided into four groups: model control group, ginsenoside Rh2 group (15 mg/kg), anti-PD-1 antibody group (200 µg/mice), and ginsenoside Rh2 in combination with anti-PD-1 antibody group. The administration time and volume were as follows: ginsenoside Rh2 was administered intragastrically 5 days before vaccination; anti-PD-1 antibody was administered intraperitoneally on days 3, 6, 9, and 12 after vaccination.

### 2.2 Vaccination

SCC-7 cells were resuscitated and expanded in large quantities. Trypsin was added to digest and the cells were collected. After counted, the cells were re-suspended in PBS solution and the cell concentration was adjusted, and the mice were inoculated with 5*10⁶ cells/0.2 mL in the right armpit.

### 2.3 Tumor growth monitoring

After tumor inoculation, the growth status of the transplanted tumors was monitored every day, and the effects of tumor growth and medicament treatment on the normal behavior and weight of the mice were observed. Throughout the experiment, the tumor volume of the mice was measured twice weekly.

### 2.4 Collection of materials

After the experiment, the mice's eyeballs were removed to collect blood, and the tumor tissue was peeled off. The tumor tissue was divided into two parts, one part was quickly frozen in liquid nitrogen and then transferred to a -80°C refrigerator for storage, and the other part was stored in PBS solution for flow cytometry detection.

### 2.5 Detection of tumor-infiltrating lymphocyte levels in mice

Mice tumor samples were cut into pieces with sterile scissors, ground gently with a syringe stopper, and filtered through a 40 µm filter to prepare a single cell suspension. The cell suspension was transferred to a new centrifuge tube, PBS solution was added, the supernatant was discarded after centrifugation, and the remaining was washed with PBS and centrifuged again. PBS was added to re-suspended, 100 µL of resuspending solutionwas placed into the flow tube, the corresponding flow cytometry antibody was added for incubating and staining for 20 minutes, and then on-machine detection was performed.

### 3. Experimental result

### 3.1 Ginsenoside Rh2 can enhance the inhibitory effect of anti-PD-1 antibody on head and neck squamous cell carcinoma

As shown in Figure 6, the tumor volume of the mice in the model control group continued to increase over time. The use of ginsenoside Rh2 alone could significantly reduce the tumor size of the mice. The tumor volume of the mice using anti-PD-1 antibody alone decreased slightly. The combination of ginsenoside Rh2 and anti-PD-1 antibody can further reduce the tumor volume compared with ginsenoside Rh2 or anti-PD-1 antibody alone, and the difference is statistically significant (P<0.05, Figure 6A). In addition, after the experiment, the tumor tissue was peeled off and weighed. It is found that the effects of ginsenoside Rh2 and anti-PD-1 antibody on mice tumor weight were similar to the results of tumor volume (Figure 6B). The above results show that ginsenoside Rh2 can significantly promote the inhibitory effect of anti-PD-1 antibody on head and neck squamous cell carcinoma, and the combination of ginsenoside Rh2 and anti-PD-1 antibody has a synergistic effect.

### 3.2 Ginsenoside Rh2 can enhance the infiltration level of immune cells in mice tumor tissues

As shown in Figures 7A and 7D, neither ginsenoside Rh2 nor anti-PD-1 antibody had a significant effect on the number of T cells when administered alone, but their combination could further increase the abundance of T cells infiltrating into tumors. As shown in Figures 7B and 7E, for CD8⁺ T cells with a killing effect, the infiltration level of CD8⁺ T cells in the tumors of mice treated with anti-PD-1 antibody increased significantly, and the infiltration ratio was also significantly increased in combination with ginsenoside Rh2 compared to that administered alone (P<0.05). In addition, as shown in Figures 7C and 7F, ginsenoside Rh2 alone can significantly increase the proportion of B cell activation, and combination with anti-PD-1 antibody can also significantly increase it (P<0.05). Through statistical analysis of the above results, we found that compared with the use of anti-PD-1 antibody alone, the combination group increased T cells by 13%, CD8⁺ T cells by 54%, and activate B cells by 38%. The above results show that use of ginsenoside Rh2 in combination with anti-PD-1 antibody can increase the infiltration abundance of total T cells and CD8⁺ T cells in the tumor microenvironment, and promote B cell activation, thereby enhancing the anti-tumor immune response.

## Claims

1. Use of a ginsenoside in combination with a PD-1 blocker in preparing a medicament against head and neck squamous cell carcinoma.

2. Use of a ginsenoside in preparing a synergist for enhancing the therapeutic effect of the PD-1 blocker on head and neck squamous cell carcinoma.

3. A pharmaceutical composition for treating head and neck squamous cell carcinoma, wherein the pharmaceutical composition comprises a ginsenoside and a PD-1 blocker.

4. The use or pharmaceutical composition according to any one of claims 1 to 3, wherein the ginsenoside is ginsenoside Rh2 or ginsenoside Rg3.

5. The use or pharmaceutical composition according to any one of claims 1 to 4, wherein the PD-1 blocker is an anti-PD-1 antibody.

6. The use or pharmaceutical composition according to claim 5, wherein the anti-PD-1 antibody is pembrolizumab.

7. The use or pharmaceutical composition according to any one of claims 1 to 6, wherein the ginsenoside enhances the inhibitory effect of the PD-1 blocker on head and neck squamous cell carcinoma.

8. The use or pharmaceutical composition according to any one of claims 1 to 7, wherein the ginsenoside enhances the infiltration level of immune cells in tumor tissue.

9. The use or pharmaceutical composition according to any one of claims 1 to 8, wherein the ginsenoside and the PD-1 blocker enhance the killing effect of lymphocytes on tumor cells.

10. The use or pharmaceutical composition according to any one of claims 1 to 9, wherein the ginsenoside is in an oral dosage form and the PD-1 blocker is in an injection dosage form.

11. The use or pharmaceutical composition according to claim 10, the clinical dose of ginsenoside in the pharmaceutical composition is 1.5 mg/kg to 2.0 mg/kg.

12. The use or pharmaceutical composition according to any one of claims 1 to 11, wherein the pharmaceutical composition further comprises other medicament for treating or auxiliary treating head and neck squamous cell carcinoma.

13. The use or pharmaceutical composition according to claim 12, wherein the medicament for treating or auxiliary treating head and neck squamous cell carcinoma is ganoderma lucidum polysaccharide and/or astragalus polysaccharide.

14. The use or pharmaceutical composition according to any one of claims 1 to 13, wherein the pharmaceutical composition further comprises a pharmaceutically acceptable excipient.

15. A method for treating head and neck squamous cell carcinoma, comprising administering a ginsenoside and a PD-1 blocker to a subject in need thereof.

16. The method according to claim 15, wherein the ginsenoside is ginsenoside Rh2 or ginsenoside Rg3.

17. The method according to claim 15 or 16, wherein the PD-1 blocker is an anti-PD-1 antibody.

18. The method according to claim 17, wherein the anti-PD-1 antibody is pembrolizumab.

19. The method according to any one of claims 15 to 18, wherein the ginsenoside enhances the inhibitory effect of the PD-1 blocker on head and neck squamous cell carcinoma.

20. The method according to any one of claims 15 to 19, wherein the ginsenoside enhances the infiltration level of immune cells in tumor tissue.

21. The method according to any one of claims 15 to 20, wherein the ginsenoside and PD-1 blocker enhance the killing effect of lymphocytes on tumor cells.

22. The method according to any one of claims 15 to 21, wherein the ginsenoside is in an oral dosage form and the PD-1 blocker is in an injection dosage form.

23. The method according to claim 22, wherein the clinical dose of ginsenoside is 1.5 mg/kg to 2.0 mg/kg.

24. The method according to any one of claims 15 to 23, wherein the method further comprises administering other medicament for treating or auxiliary treating head and neck squamous cell carcinoma to a subject.

25. The method according to claim 24, wherein the medicament for treating or auxiliary treating head and neck squamous cell carcinoma is ganoderma lucidum polysaccharide and/or astragalus polysaccharide.
